(19) Europäisches Patentamt
European Patent Office
Office européen des brevets

(11) Veröffentlichungsnummer: **0 001 439**
**B1**

(12) **EUROPÄISCHE PATENTSCHRIFT**

(51) Int. Cl.³: **C 07 D 237/04,**
A 61 K 31/50 // C 07 D 401/04,
C 07 D 401/12, C 07 D 403/04,
C 07 D 405/04, C 07 D 409/04,
C 07 D 413/04, C 07 D 417/04,
C 07 D 471/04, C 07 D 487/04,
C 07 D 491/04, C 07 D 495/04

(21) Anmeldenummer: **78101030.1**

(22) Anmeldetag: **29.09.78**

(54) **1-N-substituierte 1.4-Dihydropyridazine, Verfahren zu ihrer Herstellung sowie ihre Verwendung für Arzneimittel.**

(30) Priorität: **10.10.77 DE 2745496**

(43) Veröffentlichungstag der Anmeldung:
**18.04.79 Patentblatt 79/8**

(45) Bekanntmachung des Hinweises auf die Patenterteilung:
**28.01.81 Patentblatt 81/4**

(84) Benannte Vertragsstaaten:
**BE CH DE FR GB NL**

(56) Entgegenhaltungen:
**Chemical Abstracts vol. 72, no. 1, 5 January 1970,**
**G. WESTPHAL "Heterocycles from levulinic acid. X. Formation of 1.4-dihydropyridazinecarboxylic acid esthers"**
**Seite 317, Nr. 3449a**

**BULL.SOC:CHIM.BELG., vol. 82, no. 5 to 6, 1973**
**R. A. DOMISSE et al**
**"N-Alkylation of 1,4-dihydropyridine derivatives"**
**Seite 441 bis 446**

(73) Patentinhaber: **BAYER AG**
**Zentralbereich Patente, Marken und Lizenzen**
**D-5090 Leverkusen 1, Bayerwerk (DE)**

(72) Erfinder: **Franckowiak, Gerhard, Dr.**
**Pahlkestrasse 17**
**D-5600 Wuppertal 1 (DE)**
(72) Erfinder: **Meyer, Horst, Dr.**
**Theodor-Heuss-Strasse 110**
**D-5600 Wuppertal 1 (DE)**
(72) Erfinder: **Bossert, Friedrich, Dr.**
**Claudiusweg 7**
**D-5600 Wuppertal 1 (DE)**
(72) Erfinder: **Heise, Arend, Dr.**
**Moebeck 50**
**D-5600 Wuppertal 1 (DE)**
(72) Erfinder: **Kazda, Stanislav, Dr.**
**Pahlkestrasse 55N**
**D-5600 Wuppertal 1 (DE)**
(72) Erfinder: **Stoepel, Kurt, Dr.**
**In den Birken 69**
**D-5600 Wuppertal 1 (DE)**
(72) Erfinder: **Towart, Robertson, Dr.**
**Claudiusweg 9**
**D-5600 Wuppertal 1 (DE)**
(72) Erfinder: **Wehinger, Egbert, Dr.**
**Donnenbergerstrasse 90**
**D-5620 Velbert 15 (DE)**

EP 0 001 439 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# 1-N-substituierte 1,4-Dihydropyridazine, Verfahren zu ihrer Herstellung sowie ihre Verwendung für Arzneimittel

Die vorliegende Erfindung betrifft neue 1-N-substituierte 1,4-Dihydropyridazine, ein Verfahren zu ihrer Herstellung sowie ihre Verwendung für Arzneimittel, insbesondere kreislaufbeeinflussende und spasmolytisch wirksame Mittel.

Es ist bereits bekannt geworden, daß man N-substituierte stickstoffhaltige Heterocyclen mit erhält, wenn man die Anionen der Heterocyclen mit Elektrophilen umsetzt (vgl. N. Lubavin, Chem. Ber. *2,* 100 (1869); A. Pictet, Chem. Ber. *37,* 2797 (1904); W. Tschelnizew und B. Marxorow, Chem. Ber. *60,* 194 (1927), H. Staudinger und Sutter, Chem. Ber. *53,* 1104 (1920); G. Ciamician, Chem. Ber. *20,* 1369 (1887)).

Die vorliengende Erfindung betrifft neue 1-N-substituierte 1.4-Dihydropyridazine der Formel (I)

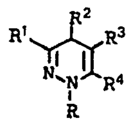

(I)

in welcher

R für geradkettiges oder verzweigtes Alkyl mit 1 bis 4 Kohlenstoffatomen steht, wobei die Alkylkette gegebenenfalls durch ein Sauerstoffatom unterbrochen ist oder wobei die Alkylkette durch Fluor oder eine Carboalkoxygruppe mit bis zu 4 Kohlenstoffatomen oder durch einen Phenylrest substituiert ist, wobei der Phenylrest gegebenenfalls ein oder zwei gleiche oder verschiedene Substituenten aus der Gruppe Fluor, Chlor, Nitro, Cyano, Trifluormethyl, Trifluormethoxy, Methoxy oder Dimethylamino trägt oder
für die Gruppe $COR^5$ steht, wobei

$R^5$ einen geradkettigen oder verzweigten Alkylrest mit 1 bis 4 Kohlenstoffatomen bedeutet, der gegebenenfalls durch ein Sauerstoffatom unterbrochen oder durch Fluor oder Phenyl substituiert ist,
oder wobei

$R^5$ für einen Phenyl- oder Benzylrest steht, der gegebenenfalls durch Methyl, Methoxy, Halogen, Nitro, Cyano oder Trifluormethyl substituiert ist
oder wobei

$R^5$ für die Gruppe $OR^6$ steht, in welcher

$R^6$ für geradkettiges oder verzweigtes Alkyl mit 1 bis 4 Kohlenstoffatomen steht, welches gegebenenfalls durch ein Sauerstoffatom unterbrochen ist oder welches gegebenenfalls durch Fluor oder eine Dimethylaminogruppe substituiert ist
oder

$R^6$ für einen Phenyl- oder Benzylrest steht, der gegebenenfalls durch Methyl, Methoxy, Halogen, Nitro, Cyano, Trifluoromethoxy, Trifluormethyl oder Dimethylamino substituiert ist,

$R^1$ für Wasserstoff oder geradkettiges oder verzweigtes Alkyl mit 1 bis 4 Kohlenstoffatomen steht, wobei die Alkylkette gegebenenfalls durch ein Sauerstoffatom unterbrochen ist oder wobei der Alkylrest substituiert ist durch Halogen, insbesondere Fluor,

$R^2$ für einen Phenylrest steht, der gegebenenfalls substituiert ist durch Halogen, Nitro, Cyano, Trifluormethyl, Trifluormethoxy, Alkyl oder Alkoxy mit 1 bis 2 Kohlenstoffatomen, oder für einen Pyridyl-, Thienyl-oder Furylrest steht,

$R^3$ für die Gruppe $COR^5$ oder die Gruppe $COOR^6$ steht, wobei $R^5$ und $R^6$ jeweils für geradkettiges oder verzweigtes Alkyl mit 1 bis 4 Kohlenstoffatomen stehen, wobei die Alkylkette gegebenenfalls durch ein Sauerstoffatom unterbrochen ist
oder

$R^5$ gemeinsam mit dem Substituenten $R^4$ einen 5- bis 6-gliedrigen carbocyclischen Ring bildet, der gegebenenfalls noch ein Sauerstoffatom enthält,

$R^4$ für einen Alkylrest mit 1 bis 2 Kohlenstoffatomen steht, der gegebenenfalls mit $R^5$ den oben erwähnten Ring bildet.

Es wurde gefunden, daß man die neuen 1-N-substituierten 1.4-Dihydropyridazine erhält, wenn man 1.4-Dihydropyridazine der Formel (II)

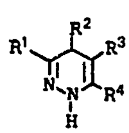

(II)

in welcher

$R^1$, $R^2$, $R^3$ und $R^4$ die oben angegebene Bedeutung haben, zunächst mit einer Base der Formel (III)

$$B^{\ominus} \qquad (III)$$

wobei

$B^{\ominus}$ für starke Basen wie Alkalihydroxide, Alkalialkoholate, Alkali- und Erdalkalihydride, Alkaliamide, Alkalidialkylamide und Alkalialkyle steht,

in ein Anion überführt und dieses dann mit einer elektrophilen Verbindung der Formel (IV)

$$R—X \qquad (IV)$$

worin

R die oben angegebene Bedeutung hat und

X für einen austretenden Rest wie Halogen oder Dialkyloxonium-, Dialkylsulfonium- oder Trialkylammoniumrest oder für einen Aryl- oder Trifluormethylsulfonsäurerest steht,

in geeigneten inerten organischen Lösungsmitteln umsetzt.

Die neuen erfindungsgemäßen 1-N-substituierten 1.4-Dihydropyridazine besitzen wertvolle pharmakologische Eigenschaften. Aufgrund ihrer Kreislaufbeeinflussenden und spasmolytischen Wirkungen können sie als antihypertensive Mittel, als Vasodilatatoren sowie als Coronartherapeutika Verwendung finden. Die erfindungsgemäßen Verbindungen stellen eine neuartige Stoffklasse zur Kreislaufbehandlung dar und sind somit als eine Bereicherung der Pharmazie anzusehen.

Verwendet man 3-Ethyl-1.4-dihydro-6-methyl-4-(3′-nitrophenyl)-pyridazin-5-carbonsäureethylester, Natriumhydrid und Chlormethyl-ethylether als Ausgangsstoffe, so kann der Reaktionsablauf durch das folgende Formelschema beispielhaft wiedergegeben werden:

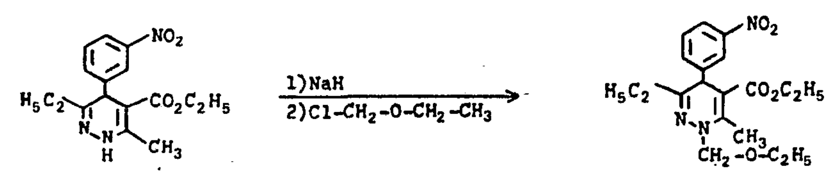


Gemäß der angegebenen Verfahrensweise wird ein 1.4-Dihydropyridazin der Formel (II) zunächst mit einer geeigneten Base der Formel (III) in das entsprechende Anion überführt und im folgenden mit einem Elektrophil der Formel (IV) zu einem 1-N-substituierten 1.4-Dihydropyridazin der Formel (I) umgesetzt.

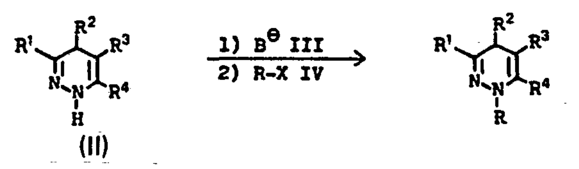


(II)

Die als Ausgangsstoffe verwendeten 1.4-Dihydropyridazine sind literaturbekannt oder können nach literaturbekannten Methoden hergestellt werden (vgl. C. Paal und C. Koch, Chem. Ber. *36*, 499 ff und 2538 ff (1903); W. J. Hale, J. Amer. Chem. Soc. *38*, 2833 (1916)).

Die als Ausgangsstoffe verwendeten Basen der Formel (III) sind literaturbekannt und handelsüblich (vgl. L. Fieser und M. Fieser, Reagents for Organic Synthesis, Vol. 5, John Wiley und Sons, New York, N.Y. 1975).

Als Beispiele seien genannt:

Natriumhydroxid, Kaliumhydroxid, Natriummethylat, Kalium-t-butylat, Natriumhydrid, Natriumamid, Kaliumhydrid, Lithiumbis- (trimethylsilyl)amid, Lithiumdiisopropylamid, Lithiumisopropylcyclohexylamid, Methyllithium und n-Butyllithium.

In der Formel (IV) hat R die oben angegebene Bedeutung und X steht vorzugsweise für einen austretenden Rest wie Halogen, insbesondere Chlor, Brom oder Jod, oder einen Dialkyloxonium-, Dialkylsulfonium- oder Trialkylammoniumrest mit insbesondere 1 bis 4 Kohlenstoffatomen je Alkylgruppe oder für den p-Toluolsulfonsäure- oder Trifluormethylsulfonsäurerest.

Die als Ausgangsstoffe verwendeten Elektrophilen sind teils literaturbekannt oder können nach literaturbekannten Methoden hergestellt werden (vgl. L. Fieser und M. Fieser, Reagents for Organic Synthesis, Vol. I und II, John Wiley & Sons, New York, N.Y., 1967).

Als Beispiele seien genannt:

Ethylbromid, Ethyljodid, n-Proöyljodid, i-Propyljodid, n-Butylbromid, sek.-Butylbromid, i-Butyljodid, Chlormethylethylether, Perfluorethyljodid, Cyclopentylbromid, Trethyloxoniumtetrafluoroborat, Proparyl-

bromid, Allylbromid, Benzylbromid, Benzylchlorid, p-Benzyloxybenzylchlorid, m-, p-Chlorbenzylbromid, 3,4-Dichlorbenzylbromid, o-, m-, p-Nitrobenzylbromid, 2-Methoxy-4-nitrobenzylbromid, 4-Trifluormethylbenzylchlorid, Bromessigsäuremethylester, Chloressigsäureethylester, 2-Brompropionsäureethylester, 3-Brom-propionsäuremethylester, Bromessigsäurehexylester, Acetylchlorid, Acetylbromid, Propionylchlorid, n-Butyrylchlorid, Cyclohexancarbonsäurechlorid, Crotonylchlorid, Methoxyacetylchlorid, Hexanoylchlorid, Phenylacetylchlorid, m-Nitrophenylacetylbromid, Benzoylchlorid, m-Nitrobenzoylchlorid, 3,4-Dichlorbenzoylchlorid, 4-Cyanobenzoylchlorid, m-Trifluormethylbenzoylchlorid, m-Trifluormethoxybenzoylchlorid, p-Dimethylaminobenzoylchlorid, p-Fluorbenzylchlorid, m-, p-, o-Toluylchlorid, 2-Furoylchlorid, Thiophen-2-carbonsaäurechlorid, Isonicotinsäurechlorid, Nicotinsäurechlorid, Picolinsäurechlorid, Zimtsäurechlorid, m-Chlorzimtsäurechlorid, Chlorameisensäuremethylester, Chlorameisensäureethylester, Chlorameisensäure-n-butylester, Chlorameisensäurei-butylester, Chlorameisensäurebenzylester, Chlorameisensäurephenylester, Chlorameisensäure-(4-nitrophenyl)-ester, Chlorameisensäureperfluorethylester, Chlorameisensäure-p-methoxyphenylester.

Als Verdünnungsmittel kommen alle inerten organischen Lösungsmittel in Frage. Hierzu gehören vorzugsweise Ether wie Dioxan, Diethylether, Tetrahydrofuran, Glykoldimethylether oder Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen —78°C und 100°C, vorzugsweise im Bereich von —20 bis +20°C.

Die Umsetzung kann in inerter Atmosphäre bei Normaldruck, aber auch bei erhöhtem Druck durchgeführt werden. Im allgemeinen arbeitet man bei Normaldruck unter Schutzgas wie reinstem Stickstoff oder Argon.

Bei der Durchführung des erfindungsgemäßen Verfahrens wird 1 Mol des 1.4-Dihydropyridazins der Formel (II) in einem geeigneten Lösungsmittel mit 1 Mol Base der Formel (III) zur Reaktion gebracht. Nach vollständiger Umsetzung wird für den zweiten Reaktionsschritt das Elektrophil der Formel (IV) in Verdünnung zugefügt und bei geeigneter Reaktionsführung die Umsetzung vervollständigt.

Die Isolierung und Reinigung der erfindungsgemäßen Substanzen erfolgt vorzugsweise derart, daß man gegebenenfalls nach Abtrennung unlöslicher Stoffe das Lösungsmittel im Vakuum abdestilliert und den gegebenenfalls erst nach Eiskühlung kristallin erhaltenen Rüchstand aus einem geeigneten Lösungsmittel umkristallisiert.

Das vorstehende Herstellungsverfahren ist lediglich zur Verdeutlichung angegeben, und die Herstellung der Verbindungen der Formel (I) ist nicht auf dieses Verfahren beschränkt, sondern jede Modifikation dieser Verfahren ist in gleicher Weise für die Herstellung der erfindungsgemäßen Verbindungen anwendbar.

Je nach der Wahl der Ausgangssubstanzen können die erfindungsgemäßen Verbindungen in stereosomeren Formen existieren, die sich entweder wei Bild und Spiegelbild (Enantiomere) oder die sich nicht wie Bild und Spiegelbild (Diastereomere) verhalten. Sowohl die Antipoden als auch die Racemformen als auch die Diastereomerengemische sind Gegenstand der vorliegenden Erfindung Die Racemformen lassen sich ebenso wie die Diastereomeren in bekannter Weise in die stereoisomer einheitlichen Bestandteile trennen (vgl. z.B. E. L. Eliel, Stereochemistry of Carbon Compounds, Mc Graw Hill, 1962).

Außer den unten aufgeführten Herstellungsbeispielen seien folgende erfindungsgemäßen Wirkstoffe genannt:

1,3 - Diethyl - 1.4 - dihydro - 6 - methyl - 4 - (3′ - nitrophenyl) - pyridazin - 5 - carbonsäureethylester,

3 - Ethyl - 4 - (3′ - chlorphenyl) - 1.4 - dihydro - 1,6 - dimethylpyridazin - 5 - carbonsäureethylester,

3 - Ethyl - 4 - (2′ - chlorphenyl) - 1.4 - dihydro - 1 - isopropyl - 6 - methylpyridazin - 5 - carbonsäuremethylester,

3 - Ethyl - 1 - cyclopentyl - 1.4 - dihydro - 4 - (3′ - methoxyphenyl) - 6 - methylpyridazin - 5 - carbonsäureethylester,

1 - Ethoxymethyl - 3 - ethyl - 1.4 - dihydro - 6 - methyl - 4 - (2′ - trifluormethylphenyl) - pyridazin - 5 - carbonsäure - i - propylester,

3 - Ethyl - 1.4 - dihydro - 6 - methyl - 4 - (3′ - nitrophenyl) - 1 - (3′,3′,3′ - trifluorpropyl) - pyridazin - pyridazin - 5 - carbonsäureethylester,

3 - Ethyl - 1 - carboethoxymethyl - 1.4 - dihydro - 6 - methyl - 4 - (3′ - nitrophenyl) - pyridazin - 5 - carbonsäure - (β - methoxyethyl) - ester,

3 - Ethyl - 1.4 - dihydro - 6 - methyl - 4 - (3′ - nitrophenyl) - 1 - phenylacetyl - pyridazin - 5 - carbonsäureethylester,

3 - Ethyl - 1.4 - dihydro - 6 - methyl - 4 - (3′ - nitrophenyl) - 1 - (4′ - trifluormethylphenylacetyl) - pyridazin - 5 - carbonsäureethylester,

3 - Ethyl - 1.4 - dihydro - 6 - methyl - 4 - (3′ - nitrophenyl) - 1 - (3′ - nitrobenzoyl) - pyridazin - 5 - carbonsäure - t - butylester,

3 - Ethyl - 1.4 - dihydro - 1,6 - dimethyl - 4 - (3′ - nitrophenyl) - pyridazin - 5 - carbonsäure - (β - n - butoxyethyl) - ester,

3 - Ethyl - 4 - (3' - cyanophenyl) - 1.4 - dihydro - 1 - n - propyl - 6 - methylpyridazin - 5 - carbonsäureethylester,
3 - Ethyl - 1 - carboethoxymethyl - 1.4 - dihydro - 6 - methyl - 4 - (2' - nitrophenyl) - pyridazin - 5 - carbonsäure - n - propylester,
3 - Ethyl - 1 - carboethoxy - 1.4 - dihydro -6 - methyl - 4 - (2' - nitrophenyl) - pyridazin - 5 - carbonsäureethylester,
3 - Ethyl - 1.4 - dihydro - 6 - methyl - 4 - (3' - nitrophenyl) - 1 - (thienyl - (2) - carbonyl) - pyridazin - carbonsäureethylester,
3 - Ethyl - 1.4 - dihydro - 6 - methyl - 4 - (2' - nitrophenyl) - 1 - (pyridyl - (2) - carbonyl) - pyridazin - 5 - carbonsäure - ($\beta$ - methoxyethyl) - ester,
1.4 - Dihydro - 3,6 - dimethyl - 1 - (furyl - (2) - carbonyl) - 4 - (4' - trifluormethoxyphenyl) - pyridazinn - 5 - carbonsäure - i - propylester,
3 - Ethyl - 1 - cinnamoyl - 1.4 - dihydro - 6 - methyl - 4 - (2' - trifluormethylphenyl) - pyridazin - 5 - carbonsäurebenzylester,
1 - Carboethoxypropyl - 1.4 - dihydro - 6 - methyl - 4 - (3' - nitrophenyl - 3 - (thienyl - (2)) - pyridazin - 5 - carbonsäuremethylester,
1 - Carbobenzyloxy - 1.4 - dihydro - 3,4 - dimethyl - 6 - methyl - pyridazin - 5 - carbonsäureethylester,
1 - Carbophenoxy - 3 - cyclohexyl - 1.4 - dihydro - 6 - methyl - 4 - (4' - trifluormethylphenyl) - pyridazin - 5 - carbonsäure - i) - propylester,
3,4 - Di - (3' - chlorphenyl) - 1.4 - dihydro - 1.6 - dimethyl - pyridazin - 5 - carbonsäuremethylester,
1 - Carboethoxymethyl - 1.4 - dihydro - 3 - (furyl - (2)) - 6 - methyl - 4 - (2' - nitrophenyl) - pyridazin - 5 - carbonsäureethylester,
1 - Carboethoxy - 3,6 - diethyl - 1.4 - dihydro - 4 - (2' - trifluormethylphenylpyridazin - 5 - carbonsäureethylester,
3 - ($\beta$ - Ethoxyethyl) - 1 - carbobenzyloxy - 1.4 - dihydro - 6 - methyl - 4 - (2' - trifluormethylphenyl) - pyridazin - 5 - carbonsäure - n - propylester,

Die neuen Verbindungen haben ein breites und vielseitiges pharmakologisches Wirkungsspektrum.

Im einzelnen konnten im Tierexperiment folgende Hauptwirkungen nachgewiesen werden:

1) Die Verbindungen bewirken bei parenteraler, oraler und perlingualer Zugabe eine deutliche und langanhaltende Erweiterung der Coronargefäße. Diese Wirkung auf die Coronargefäße wird durch einen gleichzeitigen Nitrit-ähnlichen herzentlastenden Effekt verstärkt. Sie beeinflussen zw. verändern den Herzstoffwechsel im Sinne einer Energieersparnis.
2) Die Erregbarkeit des Reizbildungs- und Erregungsleitungssystems innerhalb des Herzens wird herabgesetzt, so daß eine in therapeutischen Dosen nachweisbare Antiflimmerwirkung resultiert.
3) Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z.B. dem Zentralnervensystem) manifestieren.
4) Die Verbindungen senken den Blutdruck von normotonen und hypertonen Tieren und können somit als antihypertensive Mittel verwendet werden.
5) Die Verbindungen haben stark muskulär-spasmolytische Wirkungen, dies an der glatten Muskulatur des Magen-Darmtraktes, des urogenitaltraktes und des Respirationssystems deutlich werden.

Die neuen Wirkstoffe können in bekannter Weise in die üblichen Formulierungen übergeführt werden, wei Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien besipielhaft aufgeführt:

Wasser, nicht-toxische organische Lösungsmittel, wie Paraffine (z.B. Erdölfraktionen), pflanzliche Öle (z.B. Erdnuß-/Sesamöl), Alkohole (z.B. Ethylalkohol, Glycerin), Glykole, z.B. Propylenglykol, Polyethylenglykol), feste Trägerstoffe, wie z.B. natürliche Gesteinsmehle (z.B. Kaoline, Tonerden, Talkum, Kreide), synthetische Gesteinnsmehle (z.B. hochdisperse Kieselsäure, Silikate), Zucker (z.B. Roh-, Milch- und Traubenzucker), Emulgiermittel, wie nicht-ionogene anionische Emulgatoren (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B.

Magnesiumstearat, Talkum, Stearinsäure und Natriumlaurylsulfat).

Die Applikation erfolt in üblicher Weise oral oder parenteral, insbesondere perlingual oder intravenös. Im Falle der oralen Anwendung können Tabletten selbstverständlich außer den genannten Trägerstoffen auch Zusätze wie Natrijmcitrat, Calciumcarbonat und Dicalciumphosphat zusammen mit verschiedenen Zuschlagstoffen, wie Stärke, vorzugsweise Kartoffelstärke, Gelatine und dergleichen, enthalten.

Weiterhin können Gleitmittel, wie Magnesiumstearat, Natriumlaurylsulfat und Talkum zum Tablettieren mitverwendet werden. Im Falle wäßriger Suspension und/oder Elixieren, die für orale Anwendung gedacht sind, können die Wirkstoffe außer mit den obengenannten Hilfsstoffen mit verschiedenen Geschmacksaufbesserern oder Farbstoffen versetzt werden. Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,05 bis 5 mg/kg Körpergewicht pro Tag zur Erzielung wirksamer Ergebnisse zu verabreichen, und bei oraler Applikation beträgt die Dosierung etwa 0,01 bis 20 mg/kg, vorzugsweise 0,1 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

*Herstellungsbeispiele*

Beispiel 1

1.4 - Dihydro - 4 - phenyl - 4,3,6, - trimethyl - pyridazin - 5 - carbonsäureethylester

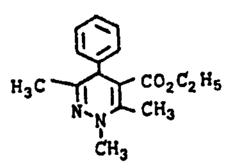

25,8 g (0,10 Mol) 1.4-Dihydro-3,6-dimethyl-4-phenyl-pyridazin-5-carbonsäuremethylester in 100 ml absol. Tetrahydrofuran werden unter Stickstoff und Feuchtigkeitsausschluß zu 3,2 g (0,12 Mol) Natriumhydrid in 20 ml absol. Tetrahydrofuran getropft und bis zum Ende der Wasserstoffentwicklung ca. 30 Minuten gerührt. Nach Abkühlen auf −15° tropft man 15,7 g (0,11 Mol) Methyljodid in 10 ml Tetrahydrofuran hinzu, rührt 1 Stunde bei −15°C, und eine weitere Stunde nach Aufwärmen auf Raumtemperatur. Der Ansatz wird dann in 1 l Wasser gegossen, das ausgefallene Öl mit Chloroform extrahiert, die organische Phase mit Wasser gewaschen, mit Natriumsulfat getrocknet und eingeengt. Der Rückstand wird in Vakuum destilliert. Siedepunkt: 181°C (0,1 Torr).

Ausbeute: 66% der Theorie.

Beispiel 2

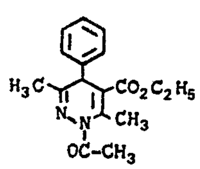

Analog Beispiel 1 wurde durch Umsetzung von 1.4-Dihydro-3,6-dimethyl-4-phenyl-pyridazin-5-carbonsäureethylester mit Lithium-diisopropylamid und Acetylchlorid in Diethylether 1-Acetyl-1-1.4-dihydro-3,6-dimethyl-4-phenyl-pyridazin-5-carbonsäureethylester vom Siedepunkt: 196°C (0,1 Torr) erhalten.

Ausbeute: 57% der Theorie.

## Beispiel 3

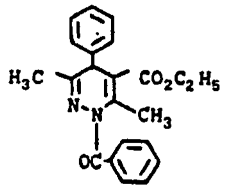

Analog Beispiel 1 wurde durch Umsetzung von 1.4-Dihydro-3,6-dimethyl-4-phenyl-pyridazin-5-carbonsäuremethylester mit Lithium-isopropylcyclohexylamid und Benzoylchlorid in Glykoldimethylether 1 - Benzoyl - 1,4 - dihydro - 3,6 - dimethyl - 4 - phenyl - pyridazin - 5 - carbonsäureethylester vom Schmelzpunkt: 115°C (Methanol) erhalten.
Ausbeute: 58% der Theorie.

## Beispiel 4

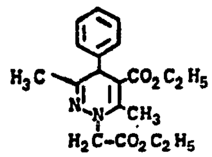

Analog Beispiel 1 wird durch Umsetzung von 1.4-Dihydro-3,6-dimethyl-4-phenyl-pyridazin-5-carbonsäureethylester mit Natriumhydrid und Bromessigsäureethylester in Dioxan 1-Carboethoxymethyl-1.4-dihydro-3,6-dimethyl-4-phenyl-pyridazin-5-carbonsäureethylester vom Siedepunkt 210°C (0,2 Torr) erhalten.
Ausbeute: 64% der Theorie.

## Beispiel 5

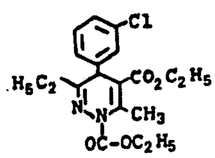

Analog Beispiel 1 wird durch Umsetzung von 3-Ethyl-4-(3'-chlorphenyl)-1,4-dihydro-6-methylpyridazin-5-carbonsäureethylester mit Natriummethylat und Chlorameisensäureethylester in Diethylether 3-Ethyl-1-carboethoxy-4-(3'-chlorphenyl)-1,4-dihydro-6-methyl-pyridazin-5-carbonsäureethylester vom Siedepunkt: 208°C (0,2 Torr) erhalten.
Ausbeute: 89% der Theorie.

## Beispiel 6

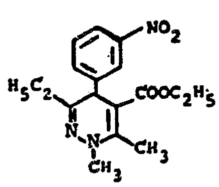

Analog Beispiel 1 wird durch Umsetzung von 3-Ethyl-1,4-dihydro-6-methyl-4-(3)nitrophenyl)-pyridazin-5-carbonsäureethylester mit Natriumamid und Methyljodid in Diethylether die Verbindung 3-Ethyl-1,4-dihydro-1,6-dimethyl-4-(3-nitrophenyl)-pyridazin-5-carbonsäureethylester vom Siedepunkt 242°C (0,1 Torr) erhalten.
Ausbeute: 68% der Theorie.

**Patentansprüche**

1. 1-N-substituierte 1,4-Dihydropyridazine der Formel (I)

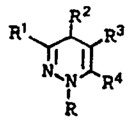

(I)

in welcher

R für geradkettiges oder verzweigtes Alkyl mit 1 bis 4 Kohlenstoffatomen steht, wobei die Alkylkette gegebenenfalls durch ein Sauerstoffatom unterbrochen ist oder wobei die Alkylkette durch Fluor oder eine Carboalkoxygruppe mit bis zu 4 Kohlenstoffatomen oder durch einen Phenylrest substituiert ist, wobei der Phenylrest gegebenenfalls ein oder zwei gleiche oder verschiedene Substituenten aus der Gruppe Fluor, Chlor, Nitro, Cyano, Trifluormethyl, Trifluormethoxy, Methoxy oder Dimethylamino trägt oder für die Gruppe $COR^5$ steht, wobei

$R^5$ einen geradkettigen oder verzweigten Alkylrest mit 1 bis 4 Kohlenstoffatomen bedeutet, der gegebenenfalls durch ein Sauerstoffatom unterbrochen oder durch Fluor oder Phenyl substituiert ist, oder wobei

$R^5$ für einen Phenyl- oder Benzylrest steht, der gegebenenfalls durch Methyl, Methoxy, Halogen, Nitro, Cyano oder Trifluormethyl substituiert ist
oder wobei

$R^5$ für die Gruppe $OR^6$ steht, in welcher

$R^6$ für geradkettiges oder verzweigtes Alkyl mit 1 bis 4 Kohlenstoffatomen steht, welches gegebenenfalls durch ein Sauerstoffatom unterbrochen ist oder welches gegebenenfalls durch Fluor oder eine Dimethylaminogruppe substituiert ist
oder

$R^6$ für einen Phenyl- oder Benzylrest steht, der gegebenenfalls durch Methyl, Methoxy, Halogen, Nitro, Cyano, Trifluoromethoxy, Trifluormethyl oder Dimethylamino substituiert ist,

$R^1$ für Wasserstoff oder geradkettiges oder verzweigtes Alkyl mit 1 bis 4 Kohlenstoffatomen steht, wobei die Alkylkette gegebenenfalls durch ein Sauerstoffatom unterbrochen ist oder wobei der Alkylrest substituiert ist durch Halogen, insbesondere Fluor,

$R^2$ für einen Phenylrest steht, der gegebenenfalls substituiert ist durch Halogen, Nitro, Cyano, Trifluormethyl, Trifluormethoxy, Alkyl oder Alkoxy mit 1 bis 2 Kohlenstoffatomen, oder für einen Pyridyl-, Thienyl-oder Furylrest steht,

$R^3$ für die Gruppe $COR^5$ oder die Gruppe $COOR^6$ steht, wobei $R^5$ und $R^6$ jeweils für geradkettiges oder verzweigtes Alkyl mit 1 bis 4 Kohlenstoffatomen stehen, wobei die Alkylkette gegebenenfalls durch ein Sauerstoffatom unterbrochen ist
oder

$R^5$ gemeinsam mit dem Substituenten $R^4$ einen 5- bis 6-gliedrigen carbocyclischen Ring bildet, der gegebenenfalls noch ein Sauerstoffatom enthält,

$R^4$ für einen Alkylrest mit 1 bis 2 Kohlenstoffatomen steht, der gegebenenfalls mit $R^5$ den oben erwähnten Ring bildet.

2. Verfahren zur Herstellung von 1-N-substituierten 1,4-Dihydropyridazinen gemäß Anspruch 1, dadurch gekennzeichnet, daß man 1,4-Dihydropyridazine der allgemeinen Formel (II)

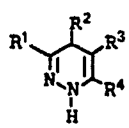

(II)

in welcher

$R^1$, $R^2$, $R^3$ und $R^4$ die inn Anspruch 1 angegebene Bedeutung haben,

zunächst mit einer Base der Formel (III)

$$B^{\ominus} \quad \text{(III)}$$

wobei

$B^{\ominus}$ für starke Basen wie Alkalihydroxide, Alkalialkoholate, Alkali- und Erdalkalihydride, Alkaliamide, Alkalidialkylamide und Alkalialkyle steht, in ein Anion überführt und dieses dann mit einer elektrophilen Verbindung der Formel (IV)

R—X (IV)

worin

R die in Anspruch 1 angegebene Bedeutung hat und

X für einen austretenden Rest wie Halogen oder Dialkyloxonium-, Dialkylsulfonium- oder Trialkylammoniumrest oder für einen Aryl- oder Trifluormethylsulfonsäurerest steht,

in geeigneten inerten organischen Lösungsmitteln umsetzt.

3. Arzneimittel enthaltend 1-N-substituierte 1,4-Dihydropyridazine gemäß Anspruch 1.

4. 1-N-substituierte 1,4-Dihydropyridazine gemäß Anspruch 1 zur Verwendung bei der Bekämpfung von Erkrankungen.

5. 1-N-substituierte 1,4-Dihydropyridazine gemäß Anspruch 1 zur Verwendung bei der Bekämpfung von Kreislauferkrankungen.

**Claims**

1. 1-N-substituted 1,4-dihydropyridazines of the formula (I)

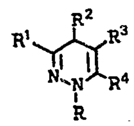

(I)

in which

R represents straight-chain or branched alkyl having from 1 to 4 carbon atoms, the alkyl chain being optionally interrupted by an oxygen atom or substituted by fluorine or a carboalkxoy group having up to 4 carbon atoms or by a phenyl radical optionally having one or two identical or different substituents from the group fluorine, chlorine, nitro, cyano, trifluoromethyl, trifluoromethoxy, methoxy or dimethylamino, or represents the group $COR^5$,
wherein

$R^5$ denotes a straight-chain or branched alkyl radical having from 1 to 4 carbon atoms which is optionally interrupted by an oxygen atom or substituted by fluorine or phenyl,
or wherein

$R^5$ represents a phenyl or benzyl radical which is optionally substituted by methyl, methoxy, halogen, nitro, cyano or trifluoromethyl,
or wherein

$R^5$ represents the group $OR^6$,
wherein $R^6$ represents straight-chain or branched alkyl having from 1 to 4 carbon atoms and optionally interrupted by an oxygen atom or optionally substituted by fluorine or a dimethylamino group or

$R^6$ represents a phenyl or benzyl radical which is optionally substituted by methyl, methoxy, halogen, nitro, cyano, trifluoromethoxy, trifluoromethyl or dimethylamino,

$R^1$ represents hydrogen or straight-chain or branched alkyl having from 1 to 4 carbon atoms, the alkyl chain being optionally interrupted by an oxygen atom or substituted by halogen, in particular fluorine,

$R^2$ represents a phenyl radical optionally substituted by halogen, nitro, cyano, trifluoromethyl, trifluoromethoxy, alkyl or alkoxy having 1 or 2 carbon atoms or it represents a pyridyl, thienyl or furyl radical,

$R^3$ represents the group $COR^5$ or the group $COOR^6$,
wherein

$R^5$ and $R^6$ each represent straight-chain or branched alkyl having from 1 to 4 carbon atoms, the alkyl chain being optionally interrupted by an oxygen atom, or $R^5$, together with the substituent $R^4$, forms a 5-membered or 6-membered carbocyclic ring which optionally contains an additional oxygen atom,

$R^4$ represents an alkyl radical having 1 or 2 carbon atoms which optionally, with $R^5$, forms the abovementioned ring.

2. A process for the production of 1-N-substituted 1,4-dihydropyridazines according to claim 1, characterised in that 1,4-dihydropyridazines of the general formula (II)

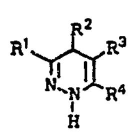

(II)

in which

$R^1$, $R^2$, $R^3$ and $R^4$ have the meaning given in claim 1 are first converted into an anion by reaction with a base of the formula (III)

$$B^{\ominus} \qquad (III)$$

wherein

$B^{\ominus}$ represents strong bases such as alkali metal hydroxides, alkali metal alcoholates, alkali metal and alkaline earth metal hydrides, alkali metal amides, alkali metal dialkyl amides and alkali metal alkyls, and the anion obtained is then reacted in suitable inert organic solvents with an electrophilic compound of the formula (IV)

$$R—X \qquad (IV)$$

wherein

R has the meaning given in claim 1 and

X represents a leaving group such as halogen or dialkyloxonium, dialkylsulphonium or trialkylammonium radical or an aryl or trifluoromethylsulphonic acid radical.

3. Medicaments containing 1-N-substituted 1,4-dihydropyridazines according to claim 1.

4. 1-N-substituted 1,4-dihydropyridazines according to claim 1 for use in combating illnesses.

5. 1-N-substituted 1,4-dihydropyridazines according to claim 1 for use in combating circulatory illnesses.

**Revendications**

1. 1,4-dihydropyridazines 1-N-substituées de formule:

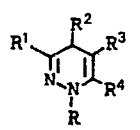

(I)

dans laquelle:

R est un groupe alkyle à chaîne droite ou ramifiée ayant 1 à 4 atomes de carbone, la chaîne alkylique étant éventuellement interrompue par un atome d'oxygène ou étant substituée par du fluor ou par un groupe carbalkoxy ayant jusqu'à 4 atomes de carbone ou par un reste phényle, le reste phényle portant éventuellement un ou deux substituants égaux ou différents du groupe fluor, chlore, nitro, cyano, trifluorométhyle, trifluorométhoxy, méthoxy ou diméthylamino ou représentant le groupe $COR^5$, dans lequel

$R^5$ est un reste alkyle à chaîne droite out ramifiée ayant 1 à 4 atomes de carbone, qui est interrompu le cas échéant par un atome d'oxygène ou par du fluor ou un groupe phényle, ou bien,

$R^5$ représente un reste phényle ou benzyle qui est substitué, le cas échéant, par un substituant méthyle, méthoxy, halogène, nitro, cyano ou trifluorométhyle ou bien $R^5$ représente le groupe $OR^6$, dans lequel

$R^6$ est un radical alkyl à chaîne droite ou ramifiée ayant 1 à 4 atomes de carbone, qui est éventuellement interrompu par un atome d'oxygène ou qui est substitué le cas échéant par du fluor ou par un groupe diméthylamino, ou bien

$R^6$ est un reste phényle ou benzyle qui est éventuellement substitué par un substituant méthyle, méthoxy, halogène, nitro, cyano, trifluorométhoxy, trifluorométhyle ou diméthylamino,

$R^1$ est un atome d'hydrogène ou un groupe alkyle à chaîne droite ou ramifiée ayant 1 à 4 atomes de carbone, dont la chaîne alkyle est éventuellement interrompue par un atome d'oxygène, ou bien le reste alkyle est substitué par un halogène, en particulier le fluor,

$R^2$ représente un reste phényle qui est substitué, le cas échéant, par un halogène ou un groupe nitro, cyano, trifluorométhyle, trifluorométhoxy, alkyle ou alkoxy ayant 1 ou 2 atomes de carbone, ou représente un reste pyridyle, thiényle ou furyle,

$R^3$ est le group de formule $COR^5$ ou le groupe de formule $COOR^6$, où $R^5$ et $R^6$ représentent chacun ou groupe alkyle à chaîne droite ou ramifiée ayant 1 à 4 atomes de carboné, la chaîne alkylique étant éventuellement interrompue par un atome d'oxygène, ou bien $R^5$ forme avec le substituant $R^4$ un noyau carbocyclique pentagonal ou hexagonal qui renferme, le cas échéant, un autre atome d'oxygène,

$R^4$ est un reste alkyle ayant 1 ou 2 atomes de carbone, qui forme, le cas échéant, avec $R^5$ le noyau mentionné ci-dessus.

2. Procédé de production de 1,4-dihydropyridazines 1-N substituées suivant la revendication 1, caractérisé en ce qu'on transforme tout d'abord en un anion des 1,4-dihydropyridazines de formule générale (II):

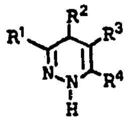

(II)

dans laquelle

$R^1$,$R^2$, $R^3$ et $R^4$ ont la définition donnée dans la revendication 1, avec une base de formule (III):

$$B^{\ominus} \qquad (III)$$

dans laquelle $B^{\ominus}$ désigne des bases fortes telles que des hydroxydes de métaux alcalins, des alcoolates de métaux alcalins, des hydrures de métaux alcalins et alcalinoterreux, des amidures de métaux alcalins, des dialkylamidures de métaux alcalins et des métaux alcalins alkylés, puis on fait réagir cet anion avec un composé électrophile de formule (IV):

$$R—X \qquad (IV)$$

dans laquelle

R a la définition indiquée dans la revendication 1, et

X est un reste partant tel qu'un halogène ou un reste dialkyloxonium, dialkylsulfonium ou trialkylammonium, ou un reste d'acide arylsulfonique ou trifluoromethyl- sulfonique,

dans des solvants organiques inertes convenables.

3. Médicament contenant des 1,4-dihydropyridazines 1-N-substituées suivant la revendication 1.

4. 1,4-dihydropyridazines 1-N-substituées suivant la revendication 1, destinées à être utilisées dans la lutte contre des maladies.

5. 1,4-dihydropyridazines 1-N-substituées suivant la revendication 1, destinées à être utilisées dans la lutte contre des troubles de la circulation.